# EUROPÄISCHE PATENTANMELDUNG

(11) **EP 4 389 178 A1**
(43) Veröffentlichungstag der Anmeldung: **26.06.2024**
(21) Anmeldenummer: 22214881.9
(22) Anmeldetag: 20.12.2022
(51) Int. Cl.: A61M 5/50, A61M 5/31

(54) **AMPULLE MIT ORIGINALITÄTSVERSCHLUSS UND VERSCHLUSSKAPPE FÜR EINE SOLCHE AMPULLE**

(71) Anmelder: Inductio AG, 4500 Solothurn (CH)
(72) Erfinder: KOLLER, Horst, 8730 Uznach (CH)
(74) Vertreter: Tergau & Walkenhorst Intellectual Property GmbH

(57) **Zusammenfassung**

Eine Ampulle (2) mit Originalitätsverschluss für eine medizinische Spritze (1), mit einem Ampullenkörper (4, 4'), der an seinem distalen Ende (6) einen Anschlussstutzen (8) zur Anbringung einer Hohlnadel oder dergleichen aufweist, soll bei einfacher Handhabung und günstiger Herstellung zuverlässig eine hohe Dichtigkeit ermöglichen. Dazu ist erfindungsgemäß eine Verschlusskappe (30, 30', 30") für den Anschlussstutzen (8) vorgesehen, wobei die Verschlusskappe (30, 30', 30") eine in einer Außenkappe (40, 70) angeordnete, mit einem an den Anschlussstutzen (8) angepassten Innenkanal (50) versehene, mit diesem auf den Anschlussstutzen (8) aufgeschobene Dichtungskappe (42, 42') aus einem vergleichsweise weichen Kunststoffmaterial umfasst, wobei die Außenkappe (40, 70) aus einem vergleichsweise harten Kunststoffmaterial gebildet ist und einen an den Ampullenkörper (4, 4') anstoßenden, in seinem Außenquerschnitt an den des Ampullenkörpers (4, 4') angepassten Kontaktring (52, 52') aufweist, und mit einem Klebeetikett (62), das zur Bildung des Originalitätsverschlusses stoffschlüssig umlaufend sowohl mit der Außenfläche (58) des Kontaktrings (52, 52') als auch in einem dem distalen Ende (6) des Ampullenkörpers (4, 4') benachbarten Mündungsbereich mit der Außenfläche des Ampullenkörpers (4, 4') verbunden ist.

## Beschreibung

Die Erfindung bezieht sich auf eine Ampulle mit Originalitätsverschluss, mit einem Ampullenkörper, der an seinem distalen Ende einen Anschlussstutzen zur Anbringung einer Hohlnadel oder dergleichen aufweist, und mit einer Verschlusskappe für den Anschlussstutzen. Sie betrifft weiter eine Verschlusskappe für eine solche Spritze.

Medikamente, insbesondere für die Behandlung in hoch spezialisierten oder komplexen Therapien, werden üblicherweise in Wirkstoff- oder Medikamentenbehältern, auch als Container oder Phiole bezeichnet, bereitgestellt. Alternativ können Medikamente aber auch in vorbefüllten Spritzen oder Ampullen bereitgestellt werden. Solche vorbefüllten Ampullen, die üblicherweise die nachfolgende Handhabung durch das Personal, insbesondere bei der Verabreichung des Wirkstoffs, deutlich erleichtern, umfassen üblicherweise den eigentlichen, mit dem Wirkstoff befüllten Ampullenkörper, an den zum Zweck der Verabreichung unmittelbar vor dem Einsatz der Spritze die Spritzennadel angebracht, beispielsweise angeschraubt oder aufgesteckt, wird. Zur Anbringung der Spitzen- oder Hohlnadel am Ampullenkörper weist dieser üblicherweise einen geeigneten Anschlussstutzen, beispielsweise als Teil einer LUER-Verbindung, auf.

Es ist allgemein wünschenswert, die bei der Verabreichung von Medikamenten verwendeten Systeme oder Komponenten für besonders geringe Material- oder Wirkstoffverluste während der gesamten Handhabungskette auszulegen. Für Behältnisse, in denen die Medikamente kurzfristig oder über einen gewissen Zeitraum hinweg vorgehalten werden, also insbesondere auch für vorbefüllte Spritzen oder vorbefüllte Ampullen, ist somit eine hohe Dichtigkeit des Verschlusssystems wünschenswert. Zu diesem Zweck ist der Anschlussstutzen einer solchen vorbefüllten Ampulle üblicherweise mit einer Verschlusskappe verschlossen, die unmittelbar vor Verwendung der Ampulle, also unmittelbar vor Anbringung der Spritzen- oder Hohlnadel, vom Anschlussstutzen entfernt wird.

Üblicherweise sind solche vorbefüllten Spritzen oder Ampullen mit einem so genannten Originalitätsverschluss versehen, der in der Art eines Siegels bei erstmaligem Gebrauch der Ampulle aufgebrochen werden muss, beispielsweise durch Abreißen von Verbindungsstegen der Verschlusskappe mit einem am Ampullenkörper verbleibenden Element wie beispielsweise einem Abreißring oder dergleichen. Durch einen solchen Originalitätsverschluss kann unter anderem sichergestellt werden, dass die Ampulle bei der Verwendung nicht schon geöffnet war und damit möglicherweise kontaminiert, sein könnte, und auch dass keine unautorisierte Manipulation des Inhalts, also des im Ampullenkörper vorgehaltenen Wirkstoffs, stattgefunden hat.

Angesichts der hohen Stückzahlen, in denen solche vorbefüllten Ampullen zum Einsatz kommen, besteht das generelle Bedürfnis, bei hoher Zuverlässigkeit und Dichtigkeit des Verschlusssystems eine besonders einfache und zuverlässige Handhabung und eine besonders kostengünstige Herstellung zu ermöglichen.

Der Erfindung liegt daher die Aufgabe zugrunde, eine Ampulle mit Originalitätsverschluss der vorstehend beschriebenen Art anzugeben, mit der bei einfacher Handhabung und günstiger Herstellung zuverlässig eine hohe Dichtigkeit erreichbar ist. Des Weiteren soll eine Verschlusskappe für eine solche Spritze angegeben werden.

Bezüglich der Ampulle wird diese Aufgabe erfindungsgemäß gelöst, indem die Verschlusskappe eine in einer Außenkappe angeordnete, mit einem an den Anschlussstutzen angepassten Innenkanal versehene, mit diesem auf den Anschlussstutzen aufgeschobene Dichtungskappe aus einem vergleichsweise weichen Kunststoffmaterial umfasst, wobei die Außenkappe aus einem vergleichsweise harten Kunststoffmaterial gebildet ist und einen an den Ampullenkörper anstoßenden, in seinem Außenquerschnitt an den des Ampullenkörpers angepassten Kontaktring aufweist, und mit einem Klebeetikett, das zur Bildung des Originalitätsverschlusses stoffschlüssig umlaufend sowohl mit der Außenfläche des Kontaktrings als auch in einem dem distalen Ende des Ampullenkörpers benachbarten Mündungsbereich mit der Außenfläche des Ampullenkörpers verbunden ist.

Vorteilhafte Ausgestaltungen der Erfindung sind Gegenstand der Unteransprüche.

Die Erfindung geht von der Überlegung aus, dass die Verschlusskappe für den Anschlussstutzen im Sinne einer hohen Zuverlässigkeit und guten Handhabbarkeit mehrkomponentig ausgeführt sein sollte, um auf diese Weise den unterschiedlichen Anforderungen besonders gut gerecht zu werden. Dabei ist einerseits die aus einem vergleichsweise weichen und somit gut verformbaren Kunststoff gebildete Dichtungskappe gezielt für eine hohe Dichtwirkung ausgelegt, die gerade auch durch die Formgebung der Dichtungskappe mit dem integrierten, in seiner Form und Geometrie an den Anschlussstutzen angepassten und somit für einen flächigen Kontakt geeigneten Innenkanal besonders begünstigt wird. Andererseits wird die mechanische Stabilität und damit Robustheit durch die die Dichtungskappe außenseitig umgebende Außenkappe bereitgestellt, die entsprechend aus einem vergleichsweise harten Kunststoff gebildet ist.

Um bei einer solchen zweikomponentigen oder 2K-Auslegung der Verschlusskappe aber auch die üblichen Anforderungen hinsichtlich Lagerfähigkeit und Robustheit bei der Handhabung sicherzustellen, sollte eine ausreichende Festigkeit der Verbindung der Verschlusskappe mit dem Ampullenkörper gewährleistet sein. Dies kann vorliegend auf besonders einfache Weise und damit mit gering gehaltenem Herstellungs- und Kostenaufwand erreicht werden, indem einerseits die Dichtungskappe durch geeignete Formgebung, Dimensionierung und Materialwahl einen Beitrag zur Festigkeit der Verbindung leistet. Dazu sollte die Dichtungskappe mit ihrem Innenkanal geeignet an den Anschlussstutzen angepasst ist, so dass bereits die aufgeschobene Dichtungskappe eine gewisse Festigkeit der Verbindung bewirkt. Zusätzlich sollte aber auch der Originalitätsverschluss derart ausgeführt sein, dass er in der Art einer Doppelfunktion zwar einerseits die gewünschte Indikation dafür liefert, ob die Ampulle unverändert und ohne Manipulationseingriff vorliegt. Andererseits sollte er aber auch gezielt einen Beitrag zur Festigkeit der Verbindung der Verschlusskappe mit dem Ampullenkörper liefern.

Um dies zu ermöglichen, ist gemäß einem Aspekt der Erfindung vorgesehen, den Originalitätsverschluss mittels eines Klebeetiketts auszuführen, das in einem distalen Endbereich des Ampullenkörpers derart stoffschlüssig (also insbesondere klebend) angebracht wird, dass es sowohl einen Randbereich der Außenkappe der Verschlusskappe als auch einen endseitigen Bereich des Außenmantels des Ampullenkörpers erfasst. Gemäß diesem als erfinderisch angesehenen Aspekt kann das Klebeetikett somit einen Dreifachnutzen entfalten: es dient zum einen in ganz herkömmlicher Weise als Informationselement, auf dem wichtige und für die Ampulle oder die darin enthaltenen Wirkstoffe charakteristische Informationen wie beispielsweise Inhalt oder Haltbarkeit angezeigt werden. Zum anderen dient es als Originalitätsverschluss, da es bei Entfernung der Verschlusskappe vom Ampullenkörper zerstört wird. Und zum Dritten dient es als Ergänzung für die Festigkeit und Stabilität der Verbindung der Verschlusskappe mit dem Ampullenkörper, da es unter anderem auch zur Weiterleitung von Haltekräften zwischen Kappe und Ampulle beitragen kann. Dementsprechend können ohne Zugeständnisse bezüglich der Leistungsfähigkeit des Verbindungssystems zwischen Verschlusskappe und Ampullenkörper die weiteren Komponenten des Verbindungssystems, beispielsweise die Kombination von Dichtungskappe und Anschlussstutzen, entsprechend einfache und damit kostensparender ausgelegt werden.

Die Verschlusskappe kann gemäß einem Aspekt der Erfindung als "echte" zweikomponentige oder 2K-Kappe ausgelegt sein und lediglich die Dichtungskappe und die Außenkappe als Komponenten umfassen. In einer solchen Ausführungsform ist die Dichtungskappe dann unmittelbar in der Außenkappe positioniert und von dieser getragen und stabilisiert; beispielsweise könnte sie darin eingesteckt oder eingerastet sein. Gemäß einem alternativen Aspekt der Erfindung kann die Dichtungskappe aber auch an sich in einer Trägerkappe angeordnet sein, die ihrerseits in der Außenkappe gehalten ist. Eine solche mehr- oder dreikomponentige Ausführung kann besonders bevorzugt und vorteilhaft sein bei vergleichsweise dicken oder groß dimensionierten Systemen. Gemäß einem Aspekt der Erfindung ist nämlich vorgesehen, dass die Außenkappe in ihren Außenabmessungen gut an die Außenabmessungen des Ampullenkörpers angepasst ist, so dass im Kontaktbereich zwischen Außenkappe und Ampullenkörper durch den in diesem Bereich vorgesehenen Kontaktring der Außenkappe einerseits und den mündungsseitigen Endbereich des Außenmantels des Ampullenkörpers andererseits eine gemeinsame Basis- oder Grundfläche gebildet wird, auf die das Klebeetikett aufgebracht werden kann. Dadurch ist sichergestellt, dass das Klebeetikett zuverlässig eine stoffschlüssige Verbindung sowohl mit dem Kontaktring der Außenkappe als auch mit dem Ampullenkörper eingeht.

Dies kann, bei geeigneter Geometrie der genannten Komponenten, insbesondere bei vergleichsweise "schmalen" Ampullenkörpern, durch ein System realisiert werden, bei dem die Dichtungskappe unmittelbar in der Außenkappe angeordnet ist. Bei größeren Außendurchmessern kann es allerdings vorteilhaft sein, unter Rückgriff auf die vorzugsweise standardisiert ausgeführte Dichtungskappe und die für "schmale" Systeme ausgelegte ursprüngliche Außenkappe, in der Art eines Adapterstücks eine weitere Außenkappe bereitzustellen, die die "ursprüngliche" Außenkappe aufnimmt und trägt, und die dann ihrerseits den Kontaktring für den Anschluss an den Ampullenkörper bereitstellt. In dieser Ausführungsform dient die "ursprüngliche" Außenkappe somit als Trägerkappe für die Dichtungskappe. Ein solches Gesamtsystem hat insbesondere den Vorteil, dass auch bei verschieden dimensionierten Ampullenkörpern grundsätzlich auf standardisierte Bauteile, nämlich die Dichtungskappe und die "ursprüngliche" Außenkappe, zurückgegriffen werden kann, so dass besonders große Stückzahlen nutzbar sind.

Die Dichtungskappe ist bevorzugt aus TPE oder Gummi gebildet. In alternativer oder zusätzlicher vorteilhafter Ausgestaltung ist weiterhin die Träger- und/oder Au-βenkappe aus Polypropylen (PP), einem Polyolefin, Cyclo-Olefin-Copolymer (COC), Cyclo-Olefine-Polymer (COP) oder Polycarbonat gefertigt.

Gemäß einem als eigenständig erfinderisch angesehenen Aspekt der Erfindung sind der Anschlussstutzen der Ampulle zu seinem freien Ende hin sich verjüngend, vorzugsweise konisch, besonders bevorzugt als LUER-Konus, und daran angepasst der Innenkanal der Dichtungskappe zu seinem freien Ende hin sich erweiternd, vorzugsweise in der Art eines invertierten Konus, ausgestaltet. Aufgrund der Selbsthemmung einer Konusverbindung, gerade auch in Kombination mit den Materialeigenschaften im Verbindungsbereich, auch unter Berücksichtigung der Eigenschaften des verformbaren weichen Kunststoffmaterials der Dichtungskappe, kann nämlich durch diese Ausgestaltung gemäß einem Aspekt der Erfindung bereits eine vergleichsweise hohe Stabilität und Festigkeit der Verbindung erreicht werden.

Die die Dichtungskappe tragende Träger- bzw. Außenkappe kann in einem den Innenkanal der Dichtungskappe umschließenden Bereich schaftartig ausgeführt sein, wobei außenseitig an diesem Schaft ein Gewinde angeordnet sein kann. Dieses kann gemäß einem Aspekt der Erfindung in ein korrespondierendes Innengewinde am Anschlussstutzen der Ampulle einschraubbar sein, beispielsweise wenn der Anschlussbereich der Ampulle in der Art einer Luer-Verbindung mit Luer-Gewinde ausgestaltet ist. In einer solchen Ausgestaltung kann die Festigkeit und Stabilität der Verbindung im Wesentlichen über diese Schraubverbindung sichergestellt werden. Gemäß einem Aspekt der Erfindung und in besonders vorteilhafter Ausgestaltung ist die die Dichtungskappe haltende Träger- bzw. Außenkappe in einem den Innenkanal der Dichtungskappe umschließenden Bereich gewindefrei ausgeführt. Damit ist einerseits die Herstellung besonders einfach gehalten, und andererseits ist ein solches System dann ohne Weiteres per se mit einer großen Vielzahl an Ausführungen des Anschlussstutzens kombinierbar.

Gemäß einem Aspekt der Erfindung ist die Ampulle mit einem Medikament oder medizinischen Wirkstoff vorbefüllt.

Bezüglich der Verschlusskappe, insbesondere für eine Ampulle der vorstehend beschriebenen Art, wird die genannte Aufgabe gelöst mit einer in einer Außenkappe angeordneten Dichtungskappe aus einem vergleichsweise weichen Kunststoffmaterial umfasst, wobei die Außenkappe aus einem vergleichsweise harten Kunststoffmaterial gebildet ist und im Bereich ihres freien Endes einen eine umlaufende Klebefläche bildenden Kontaktring aufweist. Durch eine solche Ausgestaltung kann zuverlässig beim Anbringen der Verschlusskappe am Ampullenkörper im Kontaktbereich zwischen Außenkappe und Ampullenkörper durch den in diesem Bereich vorgesehenen Kontaktring der Außenkappe einerseits und den mündungsseitigen Endbereich des Außenmantels des Ampullenkörpers andererseits eine gemeinsame Basis- oder Grundfläche gebildet werden, auf die das gemäß einem Aspekt der Erfindung vorgesehene Klebeetikett aufgebracht werden kann. Dadurch ist sichergestellt, dass das Klebeetikett zuverlässig eine stoffschlüssige Verbindung sowohl mit dem Kontaktring der Außenkappe als auch mit dem Ampullenkörper eingehen kann.

Die mit der Erfindung erzielten Vorteile bestehen insbesondere darin, dass durch die mehrkomponentige Ausgestaltung der Verschlusskappe eine Mehrzahl eigentlich konkurrierender Auslegungsziele, nämlich beispielsweise hohe Dichtigkeit, hohe Zuverlässigkeit, mechanische Belastbarkeit und/oder gute Handhabbarkeit, gleichermaßen erreicht werden können. Durch die Ausgestaltung des Originalitätsverschlusses als Klebeetikett kann dieses zudem den genannten Dreifachnutzen entfalten, nämlich einerseits als Informationselement, andererseits als Originalitätsverschluss, da es bei Entfernung der Verschlusskappe vom Ampullenkörper zerstört wird, und zum Dritten als Ergänzung für die Festigkeit und Stabilität der Verbindung der Verschlusskappe mit dem Ampullenkörper. Dementsprechend können ohne Zugeständnisse bezüglich der Leistungsfähigkeit des Verbindungssystems zwischen Verschlusskappe und Ampullenkörper die weiteren Komponenten des Verbindungssystems, beispielsweise die Kombination von Dichtungskappe und Anschlussstutzen, entsprechend einfache und damit kostensparender ausgelegt werden. Die Verschlusskappe gewährleistet die Integrität der Ampulle und den Manipulationsschutz, kann aber dennoch leicht geöffnet werden. Sie bietet zudem eine nahtlose Passform für vorgefüllte Glas- oder Polymerspritzen. Die Integrität der Containerschließung ist allgemein ein wichtiges Anliegen in der Pharmaindustrie. Dieses wird ebenso erreicht wie die ebenfalls bedeutsame Benutzerfreundlichkeit.

Ein Ausführungsbeispiel der Erfindung wird anhand einer Zeichnung näher erläutert. Darin zeigen:
- FIG. 1: eine medizinische Spritze in seitlicher Ansicht, noch ohne Originalitätsverschluss,
- FIG. 2: die medizinische Spritze nach FIG. 1 im Längsschnitt in zwei verschiedenen Schnittebenen,
- FIG. 3: eine Ampulle der Spritze nach FIG. 1 in perspektivischer Ansicht in Explosionsdarstellung,
- FIG. 4: die Ampulle nach FIG. 3 im Längsschnitt in Explosionsdarstellung,
- FIG. 5: die am distalen Ende der Ampulle nach FIG. 3 angebrachte Verschlusskappe im Längsschnitt,
- FIG. 6: die Verschlusskappe nach FIG. 5 in seitlicher Ansicht,
- FIG. 7: eine alternative Ausführungsform einer am distalen Ende der Ampulle nach FIG. 3 angebrachten Verschlusskappe im Längsschnitt,
- FIG. 8: die Verschlusskappe nach FIG. 7 in seitlicher Ansicht,
- FIG. 9: die Spritze nach FIG. 1 mit angebrachtem Originalitätsverschluss,
- FIG. 10: die Spritze nach FIG. 9 mit entfernter Verschlussklappe, und
- FIG.11: eine weitere alternative Ausführungsform einer am distalen Ende der Ampulle nach FIG. 3 angebrachten Verschlusskappe im Längsschnitt.

Gleiche Teile sind in allen Figuren mit denselben Bezugszeichen versehen.

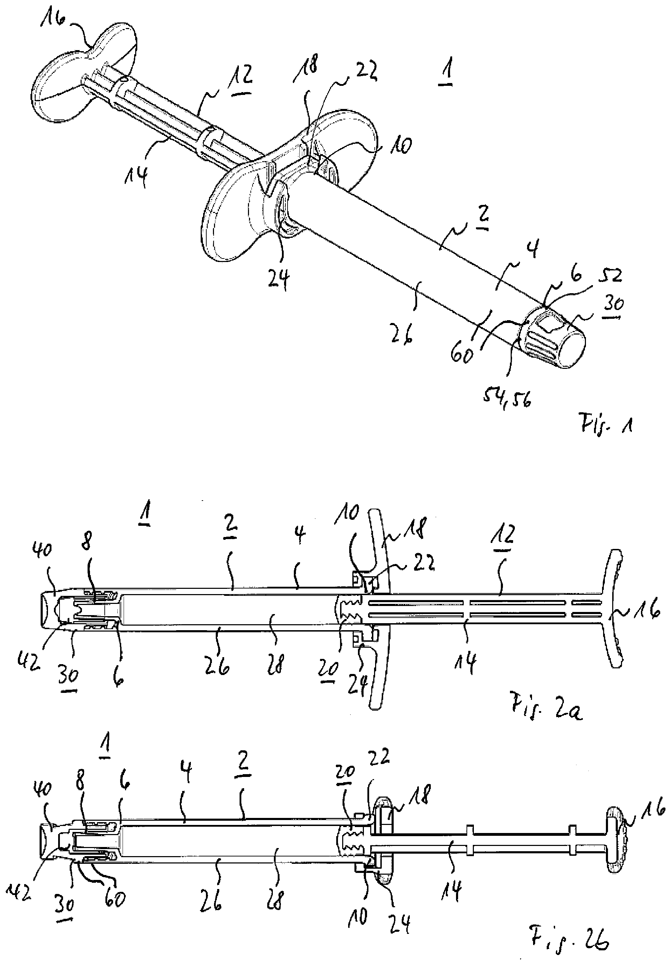

Das Spritze 1 gemäß FIGs. 1, 2 ist zur Abgabe eines medizinischen Wirkstoffs vorgesehen. Sie umfasst im Wesentlichen zwei Baugruppen, nämlich eine einen Wirkstoffbehälter bildende Ampulle 2 (auch als Karpule oder Kartusche bezeichnet) mit einem Ampullenkörper 4, der an seinem distalen Ende 6 einen Anschlussstutzen 8 zur Anbringung einer Hohlnadel oder dergleichen aufweist, und der an seinem proximalen Ende 10 mit einem Betätigungselement 12 verbunden ist. In der Gesamtansicht in FIG. 1 und auch in den Darstellungen im Längsschnitt in FIG. 2 (Fig. 2a und 2b, jeweils in unterschiedlichen Schnittebenen) ist die Spritze 1 jeweils mit einem Betätigungselement 12 dargestellt, das als gewöhnliches Drückersystem mit einem Betätigungsstößel 14 ausgeführt ist. Der Betätigungsstößel 14 ist mit einer Drückerplatte 16 versehen und über eine Grundplatte 18 an der Ampulle 2 befestigt. Der Betätigungsstößel 14 ist dabei durch die Grundplatte 18 hindurchgeführt und mit einem in der Ampulle 2 angeordneten Stopfen 20 verbunden. Alternativ sind aber auch andere Ausgestaltungen des Betätigungselements 12 denkbar; beispielweise könnte dieses als automatisiertes Betätigungselement 12 mit automatisiertem Stopfenantrieb in der Art eines Autoinjektors oder als vordosierbares Element zur Bildung eines so genannten Pen-Injektors ausgestaltet sein. Die Ampulle 2 ist dabei an ihrem proximalen Ende 10 mit einem umlaufenden Befestigungskragen 22 versehen, der zur Befestigung des Betätigungselements 12 in eine an dessen Grundplatte 18 angeformte Befestigungskulisse 24 einschiebbar ist. Gemäß einem als eigenständig erfinderisch angesehenen Aspekt ist die Ampulle 2 somit in der Art eines Plattformsystems beliebig und je nach vorgesehenem Einsatzzweck mit unterschiedlichen Bautypen des Betätigungselements 12 kombinierbar.

Die Ampulle 2 umfasst entsprechend einer herkömmlichen Bauweise einen zylindrisch oder rohrförmig den Ampullenkörper 4 bildenden Hohlkörper 26, der zur Aufnahme des medizinischen Wirkstoffs vorgesehen ist. In diesem Hohlkörper 26 ist innerhalb des die eigentliche Wirkstoffkammer bildenden Spritzenkanals 28 der Stopfen 20 geführt, der den Innenraum des Hohlkörpers 26 zu seinem proximalen Ende 10 dichtend abschließt. Zudem ist am distalen Ende 6 des Hohlkörpers 26 eine Verschlusskappe 30 angebracht, die den Innenraum des Hohlkörpers 26 dichtend abschließt. Die Ampulle 2 ist somit in ihrer Gesamtheit als abgedichteter Behälter ausgeführt, in dem der medizinische Wirkstoff für eine gewisse Lagerzeit aufbewahrt und vorgehalten werden kann. Insbesondere kann die Ampulle 2 somit als vorbefüllter Wirkstoffcontainer ausgeführt sein.

Hinsichtlich der als eigenständig erfinderisch angesehenen Materialwahl für die Ampulle 2 bzw. den diese bildenden Hohlkörper 26 ist insbesondere hohen Ansprüchen an die zuverlässige vorübergehende Lagerung des Wirkstoffs, insbesondere auch bei einer eventuell erforderlichen Lagerung in tiefgekühltem Zustand, einhergehend mit einer besonders hohen Sicherheit im Umgang mit den Komponenten Rechnung getragen. Der zylindrisch ausgeführte Hohlkörper 26 kann dabei aus COC, PC oder PP gefertigt sein, ist im gezeigten Ausführungsbeispiel aber in als erfinderisch angesehener Ausgestaltung aus dem Hochleistungskunststoff Cyclo-Olefin-Polymer (COP) gefertigt. Dieser Werkstoff zeichnet sich durch hohe Bruchfestigkeit und glasähnliche Transparenz aus. Er setzt zudem keine Alkali-Ionen frei, so dass das Risiko einer pH-Wert-Verschiebung im vorgehaltenen Wirkstoff ausgeschlossen ist.

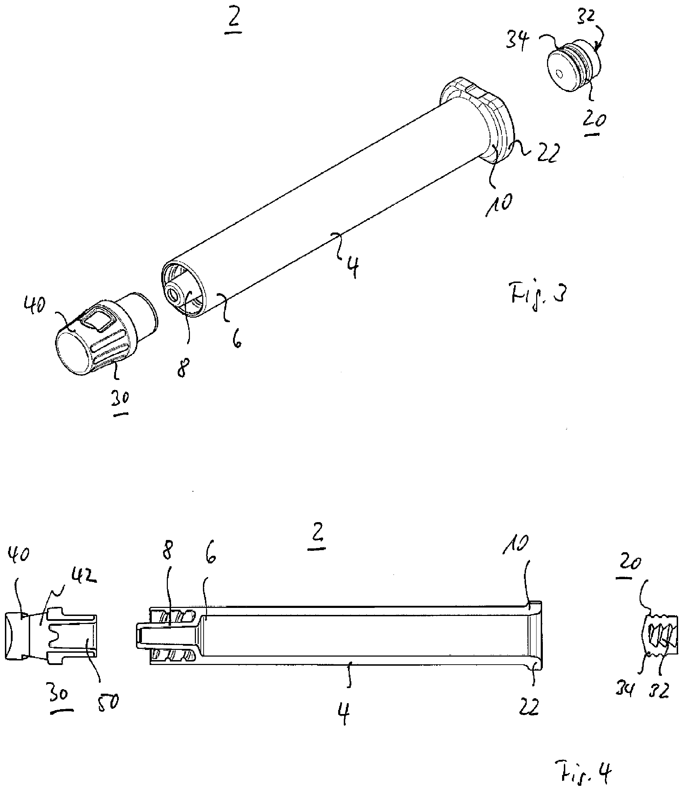

Die als eigenständig erfinderisch angesehene Ampulle 2 ist mit ihren beidseitigen Verschlusselementen, nämlich der Verschlusskappe 30 einerseits und dem Stopfen 20 andererseits, in FIG. 3 in perspektivischer Ansicht und in FIG. 4 im Längsschnitt, jeweils in Explosionsdarstellung, gezeigt. Der Ampullenkörper 4 ist dabei an seinem proximalen Ende 10 mit dem Befestigungskragen 22 versehen, um die Verbindung mit dem Betätigungselement 12 zu ermöglichen. Alternativ können natürlich auch andere Konzepte zur Befestigung des Betätigungselements 12, beispielsweise eine Schraub- oder eine Schnappverbindung, vorgesehen sein. In das proximale Ende 10 ist der Stopfen 20 einbringbar, der im eingebrachten Zustand den Ampullenkörper 4 zu seinem proximalen Ende 10 hin dichtend verschließt.

Der Stopfen 20 ist dabei über das am proximalen Ende 10 des Ampullenkörpers 4 angeordnete Betätigungselement 12 innerhalb des Ampullenkörpers 14 verschiebbar, so dass beispielsweise der im Ampullenkörper 4 vorgehaltene Wirkstoff über eine am Anschlussstutzen 8 angebrachte Hohlnadel ausgetragen und somit appliziert werden kann. Der Stopfen 20 ist dabei derart dimensioniert, dass er dichtend an der Innenwand des Ampullenkörpers 4 anliegt; im Längsbereich des Innenraums des Ampullenkörpers 4 zwischen dem distalen Ende 6 und dem Stopfen 20 wird somit das Reservoir für den Wirkstoff ausgebildet, in dem dieser vorgehalten werden kann. Bei an den Anschlussstutzen 8 angebrachter Hohlnadel ist diese medienseitig mit diesem Reservoirvolumen verbunden. Der Stopfen 20 kann innerhalb des Ampullenkörpers 4 in Richtung zum distalen Ende 6 hin verschoben werden, so dass der Wirkstoff durch die Hohlnadel aus dem Innenraum herausgedrückt wird. Dies kann insbesondere in einer silikonfrei gehaltenen Ausgestaltung sein.

Um auch höchsten Ansprüchen hinsichtlich Dichtheit einerseits und Gleiteigenschaften innerhalb des Ampullenkörpers 4 andererseits Genüge zu tun, ist der Stopfen 20 in als eigenständig erfinderisch angesehener Ausgestaltung mehrkomponentig, insbesondere zwei-komponentig, ausgeführt. Der Stopfen 20 ist dabei wie in der nicht vorveröffentlichten Europäischen Patentanmeldung Nr. 22213561.8 beschrieben ausgeführt; deren Offenbarung wird bezüglich der Ausgestaltung und der Funktionsweise des Stopfens 20 ausdrücklich in die vorliegende Offenbarung mit einbezogen ("incorporation by reference"). Insbesondere umfasst der Stopfen 20 als erste Komponente einen aus einem vergleichsweise harten Kunststoff wie beispielsweise PP, COC oder COP ausgeführten zentralen Stopfenkörper 32, der in einem als zweite Komponente vorgesehenen äußeren Dichtmantel 34 aus einem vergleichsweise weicheren Material wie beispielsweise TPE angeordnet ist.

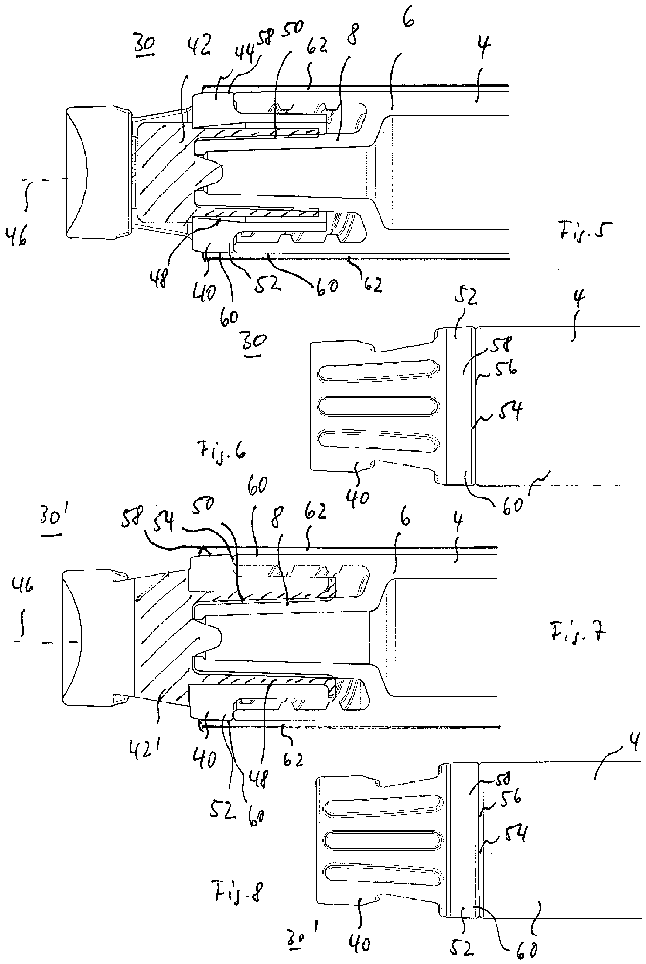

Die am Ampullenkörper 4 angebrachte Verschlusskappe 30 umfasst, wie der vergrößerten Darstellung in FIG. 5 im Längsschnitt und in FIG. 6 in seitlicher Ansicht entnehmbar ist, als wesentliche Komponenten einerseits eine starre Außenkappe 40 und andererseits eine Dichtungskappe 42 aus einem im Vergleich zur Außenkappe 40 weicheren und besser verformbaren Material, nämlich im hier dargestellten Fall aus Gummi. Die Außenkappe 40 wird von einem Grundkörper 44 gebildet, der seinerseits im Ausführungsbeispiel aus einem Kunststoff, insbesondere aus Polypropylen (PP), einem Polyolefin, Cyclo-Olefin-Copolymer (COC), Cyclo-Olefine-Polymer (COP) oder Polycarbonat, jeweils insbesondere in der Qualitätsstufe "medical grade" gefertigt ist. In diesen Grundkörper 44 ist ein sich in der durch die Längsachse 46 angedeuteten Längsrichtung erstreckender Innenkanal 48 eingearbeitet, der zur Aufnahme der Dichtungskappe 42 vorgesehen ist.

Der Grundkörper 44 der Außenkappe 40 könnte in einem "unteren", zur Herstellung der Verbindung mit dem Anschlussstutzen 8 der Ampulle 2 vorgesehen Bereich mit einem - insbesondere im Hinblick auf gängige Verbindungssysteme im Bereich medizinischer Gerätschaften vorzugsweise als Luer-Gewinde ausgestalteten - Außengewinde versehen sein. Im gezeigten Ausführungsbeispiel ist dieser Bereich jedoch gewindefrei ausgestaltet.

Die Dichtungskappe 42 ist in ihrer Außenkontur hinsichtlich Dimensionierung und Geometriewahl an den Innenkanal 48 im Grundkörper 44 der Außenkappe 40 angepasst, so dass sie in diesen einschiebbar ist und dort fixiert werden kann. In ihrem Inneren weist sie hingegen ihrerseits einen Innenkanal 50 auf, in den die Mündungskontur des Anschlussstutzens 8 der Ampulle 2 einschiebbar ist. Gemäß einem Aspekt der Erfindung ist der Anschlussstutzen 8 des Ampullenkörpers 4 zu seinem freien Ende hin sich verjüngend, im Ausführungsbeispiel konisch, insbesondere als LUER-Konus ausgeführt. Daran angepasst ist der Innenkanal 50 der Dichtungskappe 42 zu seinem freien Ende hin sich erweiternd, vorzugsweise in der Art eines invertierten Konus, ausgestaltet. Aufgrund der Materialwahl für die Dichtungskappe 42, nämlich im hier gezeigten Fall Gummi mit entsprechend weichen, elastischen Eigenschaften, und aufgrund der geometrischen Anpassung der Komponenten aneinander kann der Anschlussstutzen 8 des Ampullenkörpers 4 somit dichtend verschlossen werden. Die konische Ausgestaltung im Verbindungsbereich bewirkt dabei in synergistische Ergänzung, aufgrund der selbsthemmenden Eigenschaften einer Konusverbindung, eine zusätzliche Verstärkung und Festigkeit der Verbindung.

Die Verschlusskappe 30 umfasst somit als wesentliche Komponenten die in der Außenkappe 40 angeordnete, mit dem an den Anschlussstutzen 8 angepassten Innenkanal 50 versehene, mit diesem auf den Anschlussstutzen 8 aufgeschobene Dichtungskappe 42 aus einem vergleichsweise weichen Kunststoffmaterial, wobei die Außenkappe 40 aus einem vergleichsweise harten Kunststoffmaterial gebildet ist.

Die als eigenständig erfinderisch angesehene Verschlusskappe 30 ist darüber hinaus aber auch noch gezielt dafür ausgestaltet, einen Originalitätsverschluss für der nachfolgend beschriebenen Art für die Ampulle 2 ausbilden zu können. Dazu weist die Außenkappe 40 in der Art einer distalen Schürze einen an den Ampullenkörper 4 anstoßenden oder diesem zumindest unmittelbar benachbarten angeformten Kontaktring 52 auf. Dieser kann unter Bildung eines Stoßrings 54 direkt am Ampullenkörper 4 anstoßen oder aber auch unter Bildung eines umlaufenden Ringspalts 56 leicht beabstandet von diesem sein. Der Kontaktring 52 ist in seinem Außenquerschnitt an den des Ampullenkörpers 4 angepasst und bildet damit mit seiner auswärts gewandten Ringfläche 58 gemeinsam mit dem endseitigen Bereich des Außenmantels des Ampullenkörpers 4 eine zylindermantelförmige umlaufende Klebefläche 60 aus.

Diese durch den in diesem Bereich vorgesehenen Kontaktring 52 der Außenkappe 40 einerseits und den mündungsseitigen Endbereich des Außenmantels des Ampullenkörpers 4 andererseits gebildete Klebefläche 60 bildet eine gemeinsame Basis- oder Grundfläche, auf die das gemäß einem Aspekt der Erfindung zur Bildung des Originalitätsverschlusses vorgesehene Klebeetikett 62 stoffschlüssig aufgebracht werden kann. Dadurch ist sichergestellt, dass das Klebeetikett zuverlässig eine stoffschlüssige Verbindung sowohl mit dem Kontaktring der Außenkappe als auch mit dem Ampullenkörper eingehen kann. Das Klebeetikett 62 überdeckt dabei, wie dies beispielsweise in FIG. 5 im Längsschnitt deutlich erkennbar ist, den Stoßring 54 bzw. Ringspalt 56 zwischen Außenkappe 40 und Außenmantel des Ampullenkörpers 4. Zum Öffnen des solchermaßen verschlossenen Ampullenkörpers 4 muss somit das Klebeetikett 62 entweder entfernt oder im Bereich des Stoßrings 54 bzw. Ringspalts 56 zerstört werden, so dass eindeutig erkennbar ist, dass die Ampulle 2 bereits geöffnet wurde.

Eine alternative Ausführungsform einer am Ampullenkörper 4 angebrachten Verschlusskappe 30' ist in der vergrößerten Darstellung in FIG. 7 im Längsschnitt und in FIG. 8 in seitlicher Ansicht dargestellt. In dieser Variante ist, bei ansonsten gleicher Funktionalität der Komponenten, unter angepasster Geometrie die Dichtungskappe 42' aus TPE ausgeführt.

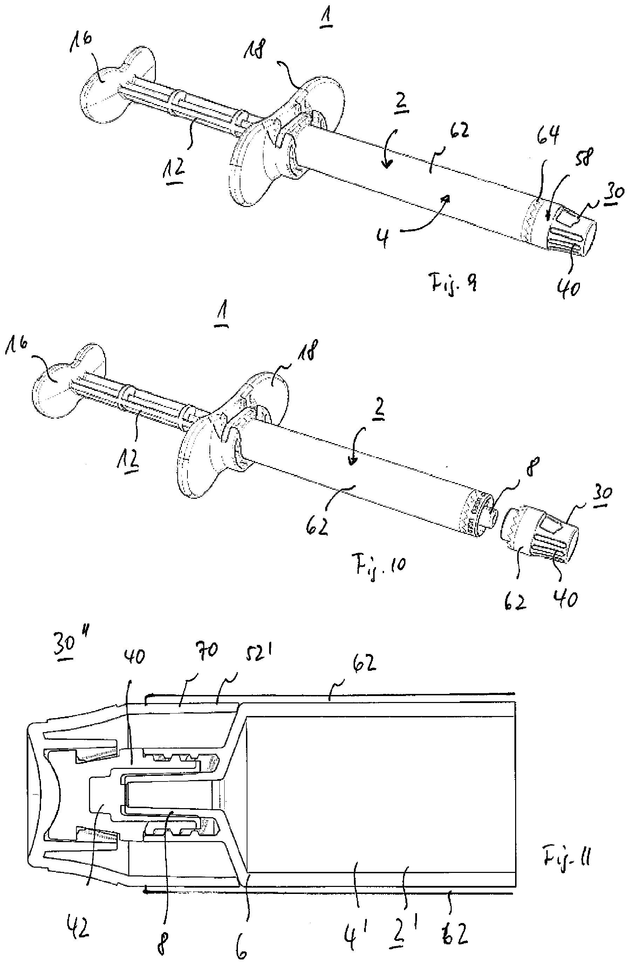

In der vorstehend beschriebenen FIG. 1 ist die Spritze 1 der besseren Verständlichkeit halber noch ohne Originalitätsverschluss dargestellt. Demgegenüber zeigt FIG. 9 die Spritze 1 nunmehr mit der Ampulle 2 mit angebrachtem Originalitätsverschluss. Deutlich erkennbar ist dabei das Klebeetikett 62, das im hier gezeigten Fall einerseits den Kontaktring 52 der Außenkappe 40 und andererseits den kompletten Außenmantel des Ampullenkörpers 4 überdeckt. Zur erleichterten Entfernung des Etiketts und damit dem Öffnen der Ampulle 2 ist das Klebeetikett 62 in seinem den Stoßring 54 bzw. Ringspalt 56 zwischen Außenkappe 40 und Ampullenkörper 4 übergreifenden Bereich mit einer Öffnungsperforation 64 in der Art einer Sollbruchstelle versehen.

Zum Öffnen der Ampulle 2 wird das Klebeetikett 62, beispielsweise durch Verdrehen der Außenkappe 40 gegenüber dem Ampullenkörper 4, aufgetrennt, wobei es beispielsweise im Bereich der Öffnungsperforation 64 aufreißt. Infolgedessen verbindet das Klebeetikett 62 die Außenkappe 40 nicht mehr mit dem Ampullenkörper 4, und die Verschlusskappe 30 kann abgenommen werden. Dies ist in FIG. 10 dargestellt. Deutlich erkennbar ist dabei, dass der Anschlussstutzen 8 der Ampulle 2 dabei frei gelegt und zugänglich gemacht wird, so dass ein Nadelkopf oder dergleichen angebracht werden kann.

Eine weitere Variante einer am distalen Ende 6 einer Ampulle 2' angebrachten Verschlusskappe 30" ist in FIG. 11 im Längsschnitt gezeigt. Hierbei handelt es sich um eine Ampulle 2' mit, in der Art eines vergrößerten Containers, im Vergleich zu den vorstehend beschriebenen Varianten vergrößertem Füllvolumen und damit vergrößertem Außendurchmesser des Ampullenkörpers 4'. Die für den Anschluss weiterer Komponenten wie beispielsweise der Spritzennadel vorgesehenen Bauteile, insbesondere der Anschlussstutzen 8, ist demgegenüber im Hinblick auf Standard-kompatible Auslegung identisch zum vorstehend beschriebenen Anschlussstutzen 8 ausgeführt, auch hinsichtlich seiner Abmessungen. Um systemübergreifend auch für solche Container im Sinne der Herstellung großer Stückzahlen unverändert die vorstehend beschriebenen Komponenten, insbesondere die Dichtungskappe 42,42' und die Außenkappe 40 weiterverwenden zu können, ist gemäß einem als eigenständig erfinderisch angesehenen Aspekt der Erfindung die Verschlusskappe 30" drei- (statt bisher zwei-) komponentig ausgeführt.

Dabei ist die Dichtungskappe 42, 42' unverändert in der Außenkappe 40 angeordnet, die in dieser Variante aber lediglich als Trägerkappe 40 dient. Diese Trägerkappe 40 ist in dieser Variante ihrerseits in einer Außenkappe 70 gehalten ist. Die Außenkappe 70 weist dabei ihrerseits den Kontaktring 52' auf, der in seinem Außendurchmesser an den des Ampullenkörpers 4' angepasst ist. Im Sinne der vorstehend beschriebenen Ausführungen kann nunmehr zur Bildung des Originalitätsverschlusses das Klebeetikett 62 stoffschlüssig sowohl mit dem Kontaktring 52' als auch mit dem Außenmantel des Ampullenkörpers 4' verbunden, insbesondere dort angeklebt werden.

### Bezugszeichenliste

- 1: Spritze
- 2: Ampulle
- 4, 4': Ampullenkörper
- 6: distales Ende
- 8: Anschlussstutzen
- 10: proximales Ende
- 12: Betätigungselement
- 14: Betätigungsstößel
- 16: Drückerplatte
- 18: Grundplatte
- 20: Stopfen
- 22: Befestigungskragen
- 24: Befestigungskulisse
- 26: Hohlkörper
- 28: Spritzenkanal
- 30, 30',: 30" Verschlusskappe
- 32: Stopfenkörper
- 34: Dichtmantel
- 40: Außenkappe
- 42,42': Dichtungskappe
- 44: Grundkörper
- 46: Längsachse
- 48, 50: Innenkanal
- 52, 52': Kontaktring
- 54: Stoßring
- 56: Ringspalt
- 58: Ringfläche
- 60: Klebefläche
- 62: Klebeetikett
- 64: Öffnungsperforation
- 70: Außenkappe

## Patentansprüche

1. Ampulle (2) mit Originalitätsverschluss für eine medizinische Spritze (1), mit einem Ampullenkörper (4, 4'), der an seinem distalen Ende (6) einen Anschlussstutzen (8) zur Anbringung einer Hohlnadel oder dergleichen aufweist, mit einer Verschlusskappe (30, 30', 30") für den Anschlussstutzen (8), wobei die Verschlusskappe (30, 30', 30") eine in einer Außenkappe (40, 70) angeordnete, mit einem an den Anschlussstutzen (8) angepassten Innenkanal (50) versehene, mit diesem auf den Anschlussstutzen (8) aufgeschobene Dichtungskappe (42, 42') aus einem vergleichsweise weichen Kunststoffmaterial umfasst, wobei die Außenkappe (40, 70) aus einem vergleichsweise harten Kunststoffmaterial gebildet ist und einen an den Ampullenkörper (4, 4') anstoßenden, in seinem Außenquerschnitt an den des Ampullenkörpers (4, 4') angepassten Kontaktring (52, 52') aufweist, und mit einem Klebeetikett (62), das zur Bildung des Originalitätsverschlusses stoffschlüssig umlaufend sowohl mit der Außenfläche (58) des Kontaktrings (52, 52') als auch in einem dem distalen Ende (6) des Ampullenkörpers (4, 4') benachbarten Mündungsbereich mit der Außenfläche des Ampullenkörpers (4, 4') verbunden ist.

2. Ampulle (2) nach Anspruch 1, deren Dichtungskappe (42, 42') in einer Trägerkappe (40) angeordnet ist, die ihrerseits in der Außenkappe (70) gehalten ist.

3. Ampulle (2) nach Anspruch 1 oder 2, deren Dichtungskappe (42, 42') aus TPE oder Gummi gebildet ist.

4. Ampulle (2) nach einem der Ansprüche 1 bis 3, deren Träger- und/oder Au-βenkappe (40, 70) aus Polypropylen (PP), einem Polyolefin, Cyclo-Olefin-Copolymer (COC), Cyclo-Olefine-Polymer (COP) oder Polycarbonat gefertigt ist.

5. Ampulle (2) nach einem der Ansprüche 1 bis 4, bei der der Anschlussstutzen (8) des Ampullenkörpers (4, 4') zu seinem freien Ende hin sich verjüngend, vorzugsweise konisch, besonders bevorzugt als LUER-Konus, und daran angepasst der Innenkanal (50) der Dichtungskappe (42, 42') zu seinem freien Ende hin sich erweiternd, vorzugsweise in der Art eines invertierten Konus, ausgestaltet sind.

6. Ampulle (2) nach einem der Ansprüche 1 bis 5, deren die Dichtungskappe (42, 42') haltende Träger- bzw. Außenkappe (40, 70) in einem den Innenkanal (50) der Dichtungskappe (42, 42') umschließenden Bereich gewindefrei ausgeführt ist.

7. Ampulle (2), die mit einem Medikament oder medizinischen Wirkstoff vorbefüllt ist.

8. Verschlusskappe (30, 30', 30"), insbesondere für eine Ampulle (2) nach einem der Ansprüche 1 bis 7, mit einer in einer Außenkappe (40, 70) angeordneten Dichtungskappe (42, 42') aus einem vergleichsweise weichen Kunststoffmaterial, wobei die Außenkappe (40, 70) aus einem vergleichsweise harten Kunststoffmaterial gebildet ist und im Bereich ihres freien Endes einen eine umlaufende Klebefläche (58) bildenden Kontaktring (52,52') aufweist.
